# EUROPEAN PATENT APPLICATION

(11) **EP 2 338 864 A1**
(43) Date of publication of application: **29.06.2011**
(21) Application number: 09180440.1
(22) Date of filing: 22.12.2009
(51) Int. Cl.: C07C 7/00, C07C 7/11, C07C 11/02

(54) **Process for removing oxygenated contaminants from an hydrocarbon stream**

(71) Applicant: Total Petrochemicals Research Feluy, 7181 Seneffe (Feluy) (BE)
(72) Inventor: Avaullee, Laurent, 6183 Trazegnies (BE); Thoret Bauchet, Jean-Pierre, 1180 Bruxelles (BE)

(57) **Abstract**

The present invention is a process for removing oxygenated contaminants and water from an hydrocarbon stream comprising :
introducing the contaminated hydrocarbon stream in a gaseous phase in an absorption zone,
contacting said hydrocarbon stream in said absorption zone at a pressure of at least 5 bars, advantageously in the range 5 to 40 bars with an alcohol capable to absorb water and oxygenated contaminants at conditions effective to produce
• an overhead hydrocarbon stream having a reduced oxygenated contaminants and water content and
• an absorbent bottoms stream comprising the alcohol, hydrocarbons and having an enhanced oxygenated contaminants and water content,

sending the overhead of the absorption zone to a wash column (referred to as the high pressure water wash column) at a pressure of at least 5 bars, advantageously in the range 5 to 40 bars, essentially washed with water at conditions effective to produce
• an overhead hydrocarbon stream having a reduced oxygenated contaminants and
• an aqueous bottoms stream having an enhanced oxygenated contaminants content.

Advantageously the process is further comprising :
sending the bottoms stream from the absorption zone to a distillation column (referred to as the alcohol distillation column) operating at a pressure of less than 3 bars absolute and advantageously at a pressure in the range 1-3 bars absolute at conditions effective to produce
an overhead comprising essentially oxygenated contaminants and hydrocarbons, optionally treated to recover the hydrocarbons,
an alcohol bottoms stream comprising water and essentially free of hydrocarbons and oxygenated contaminants,
sending said alcohol bottoms to the process which has produced the hydrocarbon stream comprising oxygenated contaminants and water to be purified.

## Description

### [Field of the invention]

The present invention is a process for removing oxygenated contaminants from an hydrocarbon stream. In a specific embodiment said hydrocarbon stream comprises olefins.

Olefins are traditionally produced from petroleum feedstocks by catalytic or steam cracking processes. These cracking processes, especially steam cracking, produce light olefin(s), such as ethylene and/or propylene, from a variety of hydrocarbon feedstock. Ethylene and propylene are important commodity petrochemicals useful in a variety of processes for making plastics and other chemical compounds.

The limited supply and increasing cost of crude oil has prompted the search for alternative processes for producing hydrocarbon products. The MTO process produces light olefins such as ethylene and propylene as well as heavy hydrocarbons such as butenes. Said MTO process is the conversion of methanol or dimethylether by contact with a molecular sieve. The interest in the methanol to olefin (MTO) process is based on the fact that methanol can be obtained from coal or natural gas by the production of synthesis gas which is then processed to produce methanol.

The effluent produced by a MTO process is a complex mixture comprising the desired light olefins, unconverted oxygenates, by-product oxygenates, heavier hydrocarbons and large amounts of water. The separation and purification of this mixture to recover the light olefins and other valuable byproducts is critical to the overall efficiency and cost effectiveness of the process. In particular, it is important that the purification scheme produces products that are substantially free of impurities, which could adversely effect downstream processing. For example, certain oxygenate components present in the effluent from an oxygenate conversion process, particularly aldehydes and ketones, may cause problems in olefin recovery operations and in derivative manufacturing processes that feed and react C₄+ hydrocarbons. There is therefore a need to ensure that the effluent purification scheme effectively removes aldehydes and ketones from the olefinic and C₄+ hydrocarbon components while at the same time minimizing loss of useful product.

Olefins can also be produced by dehydration of the corresponding alcohol. Ethanol can be obtained by fermentation of carbohydrates. Made up of organic matter from living organisms, biomass is the world's leading renewable energy source. The effluent produced by the ethanol dehydration comprises essentially unconverted ethanol, water, ethylene, acetaldehyde. Acetaldehyde may cause problems in ethylene recovery operations.

### [Background of the invention]

WO 2006-048098 A1 describes a method for removing oxygen-containing organic compounds from mixtures of various hydrocarbon compounds. A liquid phase containing hydrocarbons and oxygenates is supplied to a first column to produce a light fraction comprising the oxygenates and a bottom fraction. Said light fraction and a gaseous mixture of hydrocarbons and oxygenates are then supplied to a second column. A separation by distillation into a light and a heavy hydrocarbon fraction takes place in said second column, wherein an additional solvent is fed to the upper part of said second column which dissolves the oxygenates prior to discharging them in the bottom product of said second column. As a result, an oxygenate-free hydrocarbon product exits the head of the second column and a mixture of oxygenates, solvents and remaining hydrocarbons is removed from the bottom of the second column. The solvent can be partially or completely regenerated and recycled to the extractive distillation column. The solvent can be an alcohol such as methanol, ethanol, propanol or diethyleneglycol or N-MethylPyrrolidone (NMP). The examples are made with methanol and NMP.

US 20030045655 A1 provides a method of extracting an oxygenate from an olefin containing stream. The method comprises contacting the olefin containing stream with an extractant; and separating the contacted olefin containing stream and extractant using extractive distillation. Preferably, the extractant is a polar liquid composition at 1 atm., having an average boiling point of at least 38° C at 1 atm. More preferably, the polar liquid composition comprises at least 75 wt. % water, alcohol, or a mixture thereof. The extractants are also desirably polar compositions. Such compositions preferably contain compounds such as water, monohydric alcohols, polyhydric alcohols, or mixtures thereof. Preferred monohydric alcohols include ethanol and propanol. Preferred polyhydric alcohols include glycols. Preferred glycols include ethylene glycol and triethylene glycol. It is desirable that the extractant contains at least about 75 wt. % water, monohydric alcohol, and or polyhydric alcohol, preferably at least about 85 wt. %, more preferably at least about 90 wt. %, and most preferably at least about 95 wt. %. Water is most preferred as the extractant.

US 20030098281 A1 describes a method of controlling water and/or oxygenate concentrations of an olefin stream. The method includes contacting the olefin stream with a liquid absorbent. The liquid absorbent is selected from the group consisting of a polyol, amine, amide, nitrile, heterocyclic nitrogen containing compound, and mixtures thereof.

WO 03 020678 A2 describes a method of removing dimethyl ether from an olefin stream made from an oxygenate to olefin reaction process, comprising: contacting oxygenate with a molecular sieve catalyst to form an olefin stream, wherein the olefin stream comprises ethylene, propylene, dimethyl ether and C4+ olefin and higher boiling point hydrocarbons, separating the olefin stream into a first stream comprising the ethylene, propylene and dimethyl ether and a second stream comprising the C4+ olefin and higher boiling point hydrocarbons, and separating the dimethyl ether present in the first stream using extractive distillation. The extractants are desirably polar compositions. Such compositions preferably contain compounds such as water, monohydric alcohols, polyhydric alcohols, or mixtures thereof. Preferred monohydric alcohols include ethanol and propanol. Preferred polyhydric alcohols include glycols. Preferred glycols include ethylene glycol and triethylene glycol. It is desirable that the extractant contains at least about 75 wt.% water, monobydric alcohol, and or polyhydric alcohol, preferably at least about 85 wt.%, more preferably at least about 90 wt.%, and most preferably at least about 95 wt.%. Water is most preferred as the extractant.

WO 03 020670 A1 provides a method for removing oxygenated components such as acetaldehyde, CO2 and/or water from an olefin stream. It explains it is desirable to remove such oxygenated components, since they may poison catalysts that are used to further process olefin composition. In addition, the presence of certain oxygenated compounds, such as acetaldehyde, can cause fouling in other olefin purification units, e.g., acid gas treating units. This prior art provides a method of treating an ethylene and/or propylene containing stream. The method comprises providing an olefin stream containing ethylene, propylene, C4+ olefins and acetaldehyde. The olefin stream is separated into a first fraction and a second fraction, wherein the first fraction comprises at least a majority of the ethylene and/or propylene present in the olefin stream, and the second fraction comprises at least a majority of the C4+ olefins and acetaldehyde present in the olefin stream. The first fraction is then acid gas treated. The olefin stream is separated by distillation, preferably, the distillation is extractive distillation using an extractant. The preferred extractant is a polar composition having an average boiling point of at least 38°C at 1 atm. Methanol is one type of preferred extractant.

WO 03 020672 A1 describes method of removing dimethyl ether from an ethylene and/or propylene containing stream. The olefin stream is passed to a water absorption column, methanol is used as the water absorbent. Methanol and entrained water, as well as some oxygenated hydrocarbon, is recovered as the bottoms stream of said water absorption column, an overhead olefin is recovered and sent to a distillation column. The distillation column separates ethylene and propylene, as well as lighter boiling point components from the dimethyl ether and heavier boiling point components, including C4 + components and methanol remaining from the methanol wash. Additional methanol is added to the distillation column to reduce clathrate and/or free water formation in the distillation column. The ethylene and propylene containing stream exits the distillation column as overhead and the heavier boiling point components which include the dimethyl ether and C4 + components exit the distillation column as the bottoms. Ethylene and propylene then flow to a caustic wash column.

WO 03 033438 A1 describes a method for processing an olefin stream containing oxygenates and water, comprising: providing an olefin stream containing oxygenates and water; dewatering the olefin stream; compressing the dewatered olefin stream; washing the olefin stream with methanol to remove at least a portion of the oxygenate from the olefin stream; contacting the methanol washed olefin stream with water; and fractionating the water contacted olefin stream. The olefin stream is the effluent of an MTO process.

US 2006 258894 A1 relates to a process for extracting oxygenates from a hydrocarbon stream, typically a fraction of the condensation product of a Fischer-Tropsch reaction, while preserving the olefin content of the condensation product. The oxygenate extraction process is a liquid-liquid extraction process that takes place in an extraction column using a polar organic solvent, such as methanol, and water as the solvent, wherein the polar organic solvent and water are added separately to the extraction column.

US 2009 048474 A1 relates to a process for the production of alkene(s) from a feedstock comprising at least one monohydric aliphatic paraffinic primary (or secondary) alcohol(s), consisting of ethanol or propanol(s) or a mixture thereof, characterised by the following steps;
1. the monohydric aliphatic paraffinic primary (or secondary) alcohol(s) are converted into the corresponding same carbon number alkene(s) in a reactive distillation column at elevated pressure and temperature so that the heads stream extracted from the top of the said reactive distillation column comprises essentially the said alkene(s),
2. the heads stream from step 1 is then cooled to a temperature sufficient to condense at least part of the alkene(s) with the highest boiling point,
3. at least part of the condensed alkene(s) from step 2 are then recycled back into the said reactive distillation column, as a reflux return,
4. simultaneously the remaining alkene(s) are recovered.

### [Brief summary of the invention]

The present invention is a process for removing oxygenated contaminants and water from an hydrocarbon stream comprising :
introducing the contaminated hydrocarbon stream in a gaseous phase in an absorption zone,
contacting said hydrocarbon stream in said absorption zone at a pressure of at least 5 bars, advantageously in the range 5 to 40 bars with an alcohol capable to absorb water and oxygenated contaminants at conditions effective to produce
   - an overhead hydrocarbon stream having a reduced oxygenated contaminants and water content and
   - an absorbent bottoms stream comprising the alcohol, hydrocarbons and having an enhanced oxygenated contaminants and water content, sending the overhead of the absorption zone to a wash column (referred to as the high pressure water wash column) at a pressure of at least 5 bars, advantageously in the range 5 to 40 bars, essentially washed with water at conditions effective to produce
   - an overhead hydrocarbon stream having a reduced oxygenated contaminants and
   - an aqueous bottoms stream having an enhanced oxygenated contaminants content.

Advantageously the temperature of the absorption zone and of the high pressure water wash column is in the range 15 to 50°C and preferably in the range 15 to 40°C. Advantageously the temperature of the alcohol sent to the absorption zone is under 40°C and preferably in the range 15-40°C and more preferably in the range 15-30°C. Advantageously the temperature of the water sent to the high pressure water wash column is under 40°C and preferably in the range 15-40°C and more preferably in the range 15-30°C.

Advantageously the alcohol is an aqueous solution comprising at least 80w% of alcohol, advantageously 85w% of alcohol, more advantageously 90w% of alcohol and preferably more than 93w% of alcohol.

Optionally the overhead of the high pressure water wash column is sent to a caustic wash to remove the acidic components and recovering an hydrocarbon stream essentially free of oxygenated contaminants.

The hydrocarbon stream comprising oxygenated contaminants and water can be a stream in a refinery or a chemical plant. The hydrocarbon may comprise olefins.

In an embodiment the hydrocarbon stream comprising oxygenated contaminants and water is the effluent produced by an alcohol dehydration to make at least an olefin. By way of example the effluent produced by the ethanol dehydration comprises essentially unconverted ethanol, water, ethylene, acetaldehyde. By way of example in the ethanol dehydration the process of the present invention is very efficient to purify the ethylene. The alcohol in the absorption zone is advantageously ethanol and is capable in the ethylene stream to remove acetaldehyde as low as 15 ppm. The high pressure water wash is capable to remove ethanol in the ethylene stream as low as 20 ppm and to remove acetaldehyde as low as 10 ppm.

In another embodiment the hydrocarbon stream comprising oxygenated contaminants and water is the effluent produced by a MTO process. Said hydrocarbon stream is a complex mixture comprising the desired light olefins, unconverted oxygenates, by-product oxygenates, heavier hydrocarbons and large amounts of water. By way of example the hydrocarbon stream comprising oxygenated contaminants and water is the effluent produced by a MTO process based on methanol or dimethyl ether and the alcohol of the absorption zone is methanol.

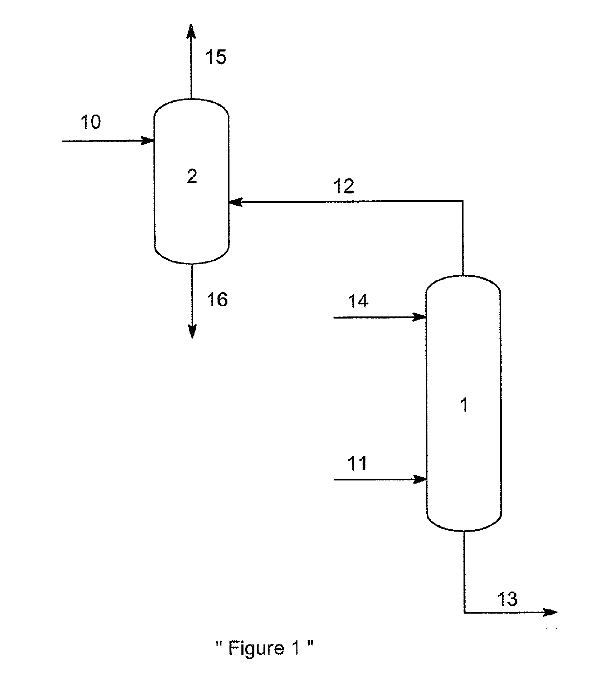

Fig 1 describes an embodiment of the invention. 1 is the absorption zone, 2 the high pressure water wash column. The hydrocarbon stream 11 comprising oxygenated contaminants and water is fed to the absorption zone 1 at 20 bars to produce an overhead hydrocarbon stream 12 having a reduced oxygenated contaminants and water content and an absorbent bottoms stream 13 comprising the absorbent, hydrocarbons and having an enhanced oxygenated contaminants and water content. The alcohol stream 14 is fed to the absorption zone 1. The overhead of the absorption zone is sent to a wash column 2 (referred to as the high pressure water wash column) at a pressure of at least 5 bars, advantageously in the range 5 to 40 bars, essentially washed with water 10 at conditions effective to produce an overhead hydrocarbon stream 15 having a reduced oxygenated contaminants and an aqueous bottoms stream 16 having an enhanced oxygenated contaminants content.

### [Detailed description of the invention]

As regards the oxygenated contaminants one can cite alcohols such as methanol, ethanol, C3 alcohols; ethers such as dimethyl ether, diethylether and methyl ethyl ether; carboxylic acids such as acetic acid, propanoic acid and butyric acid; aldehydes such as acetaldehyde; ketones such as acetone; and esters such as methyl esters. Particularly problematic oxygenate contaminants in an alcohol dehydration are aldehydes. Particularly problematic oxygenate contaminants in the MTO process are dimethyl ether (DME) and acetaldehyde.

The hydrocarbon stream comprising oxygenated contaminants and water can be available at low pressure such as 1 to 3 bars absolute and may comprise a high proportion of water. Advantageously said contaminated hydrocarbon stream is successively compressed and cooled in one or more steps to remove the major part of water and further fed to the absorption zone.

In the previous compression steps the recovered water contains a part of the oxygenated contaminants and hydrocarbons dissolved. The contaminated hydrocarbon stream can also be cooled before the first compression step and water recovered. In an embodiment the water recovered upon each cooling further to a compression step and upon cooling if any before the first compression step is sent to a stripping column to produce an overhead stream comprising essentially oxygenated contaminants and hydrocarbons and an essentially pure water bottoms stream. Optionally the overhead stream is burned to destroy the oxygenated contaminants and recover heat.

The proportion of the oxygenated contaminants in the hydrocarbon stream comprising oxygenated contaminants and water can be up to 5 w%.

Advantageously the absorbent is selected from C1 to C12 alcohols.

Olefins treated in accordance with this invention are particularly suitable for use as feedstock for making polyolefins.

Substantial amounts of water and oxygenated contaminants are removed from the hydrocarbon vapor stream by contacting the vapor stream with an effective amount of alcohol.

Conventional absorption systems can be used in this invention. In one embodiment, the absorption system uses packed columns, although plate absorption columns may also be used. In another embodiment, the absorption column has a liquid inlet located at a top portion of the absorption column. The absorbent liquid is evenly distributed across the top of the column. Desirably, an even distribution of the absorbent liquid is accomplished by using a distributor plate or spray nozzles. At the bottom of the absorption column is a gas inlet where the hydrocarbon stream, containing water and oxygenated contaminants, enters the absorption column. The vapor components move up the column countercurrent to the liquid absorbent moving down the column. This is known as countercurrent absorption. The packing or plates in the column provides a surface for intimate contact between the vapor and liquid components within the column. In a countercurrent absorption column, the concentration of soluble gasses in both the liquid and vapor phases is greatest at the bottom of the column, and lowest at the top of the column. The outlet for the liquid is at the bottom of the absorption column, typically below the gas inlet. The outlet for the gas phase lean in the gasses most soluble in the liquid absorbent is at the top of the absorption column, typically above the liquid inlet.

In an embodiment the bottoms stream of the absorption zone is sent to a distillation column operating at a pressure of less than 3 bars absolute and advantageously at a pressure in the range 1-3 bars absolute to produce (i) an alcohol bottoms stream comprising water but essentially free of oxygenated contaminants further recycled to the absorption zone and (ii) an overhead comprising essentially hydrocarbons and the oxygenated contaminants. The overhead stream of said distillation column can be treated to separate the hydrocarbons and the oxygenated contaminants. To prevent build up of water in the alcohol a purge has to be made and fresh alcohol introduced. Optionally the purge can be sent to a distillation unit to recover alcohol and the alcohol recycled in the absorption loop.

In an advantageous embodiment the alcohol of the absorption zone is further used in the process which has produced the hydrocarbon stream comprising oxygenated contaminants and water to be purified. This process is, e.g., an MTO process or an alcohol dehydration process. As regards the MTO process the alcohol is preferably methanol. By way of example the hydrocarbon stream comprising oxygenated contaminants and water is the effluent produced by a MTO process based on methanol or dimethyl ether and the alcohol of the absorption zone is methanol.

As regards an alcohol dehydration process it can be, e.g., ethanol, propanol, isobutanol. The present invention is of interest for the ethanol dehydration to produce ethylene, ethanol is therefore used to purify ethylene.

In such case of the use of the alcohol in the process further to the use as absorbent there is a huge saving in energy because the alcohol has not to be regenerated before recycling in the absorption zone. Of course the oxygenated contaminants have to be separated from the alcohol before the alcohol is sent to the process but water has not to be removed because most of time alcohol can be used in mixture with water.

In an advantageous embodiment the present invention is a process for removing oxygenated contaminants and water from an hydrocarbon stream comprising :
introducing the contaminated hydrocarbon stream in a gaseous phase in an absorption zone,
contacting said hydrocarbon stream in said absorption zone at a pressure of at least 5 bars, advantageously in the range 5 to 40 bars with an alcohol capable to absorb water and oxygenated contaminants at conditions effective to produce
   - an overhead hydrocarbon stream having a reduced oxygenated contaminants and water content and
   - an absorbent bottoms stream comprising the alcohol, hydrocarbons and having an enhanced oxygenated contaminants and water content, sending the overhead of the absorption zone to a wash column (referred to as the high pressure water wash column) at a pressure of at least 5 bars, advantageously in the range 5 to 40 bars, essentially washed with water at conditions effective to produce
   - an overhead hydrocarbon stream having a reduced oxygenated contaminants and
   - an aqueous bottoms stream having an enhanced oxygenated contaminants content,
   sending the bottoms stream from the absorption zone to a distillation column (referred to as the alcohol distillation column) operating at a pressure of less than 3 bars absolute and advantageously at a pressure in the range 1-3 bars absolute at conditions effective to produce
   - an overhead comprising essentially oxygenated contaminants and hydrocarbons, optionally treated to recover the hydrocarbons,
   - an alcohol bottoms stream comprising water and essentially free of hydrocarbons and oxygenated contaminants,
   sending said alcohol bottoms to the process which has produced the hydrocarbon stream comprising oxygenated contaminants and water to be purified.

Advantageously the overhead of the alcohol distillation column is sent to a wash column fed with water (referred to as the low pressure water wash column) to produce an overhead hydrocarbon stream comprising oxygenated contaminants and a bottoms aqueous stream comprising oxygenated contaminants. Advantageously the said bottoms of the wash column comprise the essential portion of the oxygenated contaminants to be removed. More precisely in case the contaminated hydrocarbon stream is compressed before entering the absorption zone a part of the oxygenated contaminants goes in the condensed water. The sum of the oxygenated contaminants in the said condensed water and of the oxygenated contaminants in the bottoms of the low pressure water wash column comprise the essential portion of the oxygenated contaminants to be removed, advantageously more than 90w%. Advantageously the overhead stream of the wash column comprise a small portion of the oxygenated contaminants to be removed, preferably less than about 10w%.

Optionally the said overhead of the low pressure water wash column, which contains hydrocarbons, is recycled to the process which has produced the hydrocarbon stream comprising oxygenated contaminants and water to be purified. Optionally the bottoms stream is treated to recover or destroy the oxygenated contaminants.

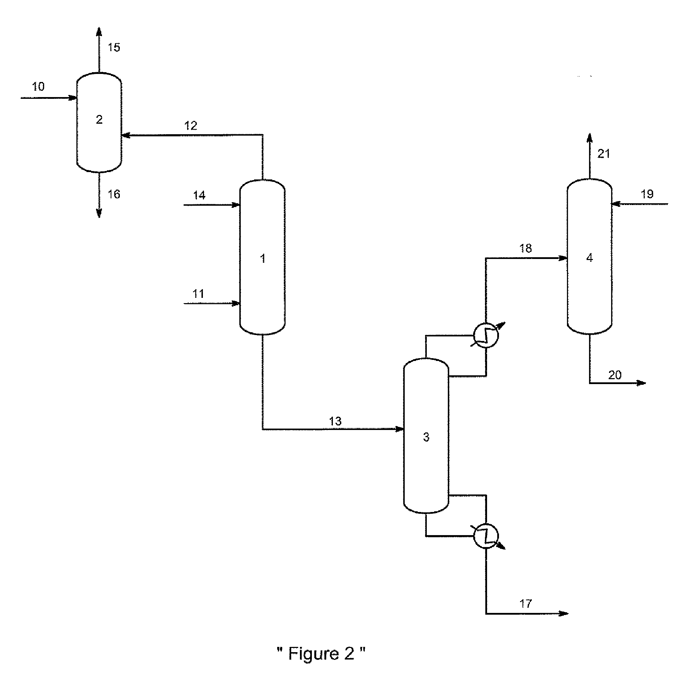

Fig 2 describes an embodiment of the invention and derives from fig 1 by incorporation of the alcohol distillation column 3 equiped with a boiler and a head condenser-separator and the low pressure water wash column 4. The bottom stream 13 from the absorption zone 1 is sent to a distillation column 3 (referred to as the alcohol distillation column) operating at a pressure of less than 3 bars absolute and advantageously at a pressure in the range 1-3 bars to produce an overhead 18 comprising essentially oxygenated contaminants and hydrocarbons, an alcohol bottoms stream 17 comprising water and essentially free of oxygenated contaminants optionally sent back to the process which has produced the hydrocarbon stream 11 comprising oxygenated contaminants and water to be purified. The overhead 18 of the alcohol distillation column 3 is sent to a wash column 4 fed with water 19 (referred to as the low pressure water wash column) to produce an overhead hydrocarbon stream 21 comprising a lower portion of oxygenated contaminants and a bottoms aqueous stream 20 comprising a higher portion of oxygenated contaminants. The overhead 21 of the wash column 4, which contains hydrocarbons, is optionally recycled to the process which has produced the hydrocarbon stream 11. Optionally the bottoms stream 20 is treated to recover or destroy the oxygenated contaminants.

The contaminated hydrocarbon stream can be available at any pressure but most of time it is at low pressure such as 1 to 3 bars absolute and may comprise a high proportion of water. MTO process and alcohol dehydration typically operate at atmospheric or low pressure in the range 1 to 3 bars absolute. In an embodiment the said hydrocarbon stream is successively compressed and cooled in one or more steps to remove the major part of water and further fed to the absorption zone. Advantageously the said hydrocarbon stream is cooled before the first compression step. In the compression steps, as well as in the cooling before the first compression step, the recovered water contains a part of the oxygenated contaminants and hydrocarbons dissolved. Typically from the initial pressure ranging from 1 to 3 bars to the pressure of the absorption zone there are about 3 to 5 compression stages. After each compression and optionally before the first compression stage water is recovered. In the course of the compression of the contaminated hydrocarbon stream to reach the pressure of the absorption zone water is condensed and the highest part is condensed at the beginning. Typically in the cooling before the first compression step and after the first compression step more than about 70% of the water contained in the contaminated hydrocarbon stream is condensed. According to the initial water content of the contaminated hydrocarbon stream the cooling before the first compression stage can produce the condensation of more than about 70% of the water. The water recovered in the course of the compression of the contaminated hydrocarbon until the entry in the absorption zone comprises :
the water condensed at the beginning (typically before the compression step and optionally after the first compression step and even optionally after the second step) and
the remaining water further condensed until entry in the absorption zone.

Advantageously the ratio of the water condensed at the beginning to the remaining water is from 40/60 to 80/20.

In an advantageous embodiment the water recovered in the course of the compression of the contaminated hydrocarbon until the entry in the absorption zone at the beginning of said course is sent to a water stripping column at low pressure, advantageously at a pressure in the range 1-3 bars absolute. Said water stripping column is equipped with a reboiler at the bottoms and a condenser separator on top and operates at conditions effective to produce
- at the condenser separator a gaseous phase comprising oxygenated contaminants and hydrocarbons, an aqueous phase comprising alcohol, oxygenated contaminants and hydrocarbons partly sent as a reflux of said stripping column, partly sent to the alcohol distillation column, optionally mixed with the bottoms from the absorption zone,
- an essentially pure water bottoms stream.

Advantageously the gaseous phase from the condenser-separator of the above water stripping column is sent to the low pressure water wash column.

Advantageously the bottoms of the high pressure water wash column are sent to the water stripping column.

Optionally the remaining part of the water condensed, after the water condensed at the beginning has been sent to the said water stripping column, is treated to recover or destroy the oxygenated contaminants.

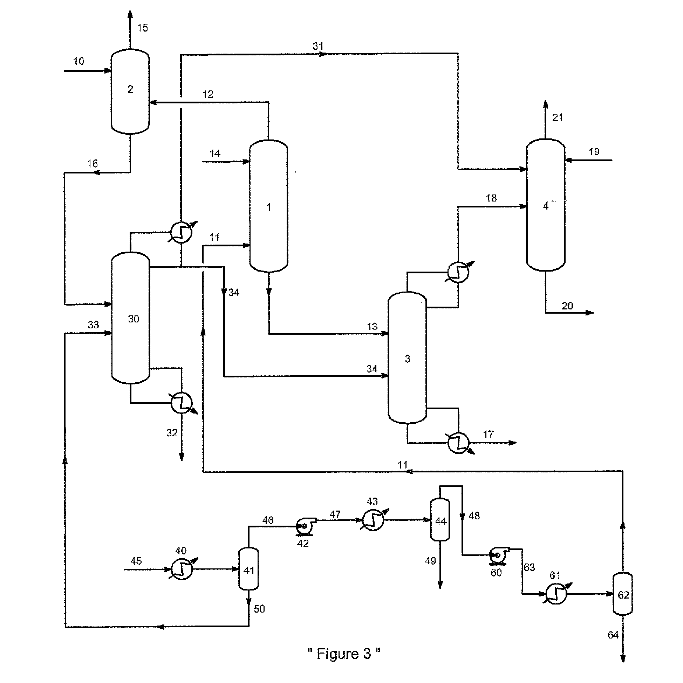

Fig 3 describes an embodiment of the invention and derives from fig 2 by incorporation of the water stripping column 30 equiped with a condenser-separator on top and a boiler at the bottoms, coolers 40, 43, 61, separators 41, 44 and 62 and compressors 42, 60. The contaminated hydrocarbon stream 45 is cooled in the cooler 40 and sent to the separator 41 to produce a water stream 50 comprising oxygenated contaminants and hydrocarbons and a gaseous stream 46. Said stream 46 is sent to the compressor 42 then to the cooler 43 and to the separator 44 to produce a water stream 49 comprising oxygenated contaminants and hydrocarbons and a gaseous stream 48. Said stream 48 is sent to the compressor 60 then to the cooler 61 and to the separator 62 to produce a water stream 64 comprising oxygenated contaminants and hydrocarbons and a gaseous stream 11 sent to the absorption zone 1. The aqueous stream 64 is treated to recover or destroy the oxygenated contaminants. Stream50 is sent as stream 33 to the water stripping column 30 to produce at the condenser separator a gaseous phase 31 comprising oxygenated contaminants and hydrocarbons, an aqueous phase comprising alcohol, oxygenated contaminants and hydrocarbons partly sent as a reflux of said stripping column, partly sent as stream 34 to the alcohol distillation column 3, optionally mixed with the bottoms 13 from the absorption zone 1. Stream 31 is sent to the low pressure water wash column 4. Bottoms 32 from column 30 are essentially pure water bottoms stream. The bottoms 16 of the high pressure water wash column 2 are sent to the water stripping column 30. Streams 32 and 49 can optionally be reused or eliminated.

Advantageously the remaining part of the water condensed in the course of the compression of the contaminated hydrocarbon stream to be fed in the absorption zone, after the water condensed at the beginning has been sent to the said water stripping column, is flashed in a flash drum. The overhead of said flash drum is sent to the low pressure water wash column and a part of the liquid phase is used as the wash water in the low pressure water wash column and in the high pressure water wash column, the remaining part of said liquid phase is purged.

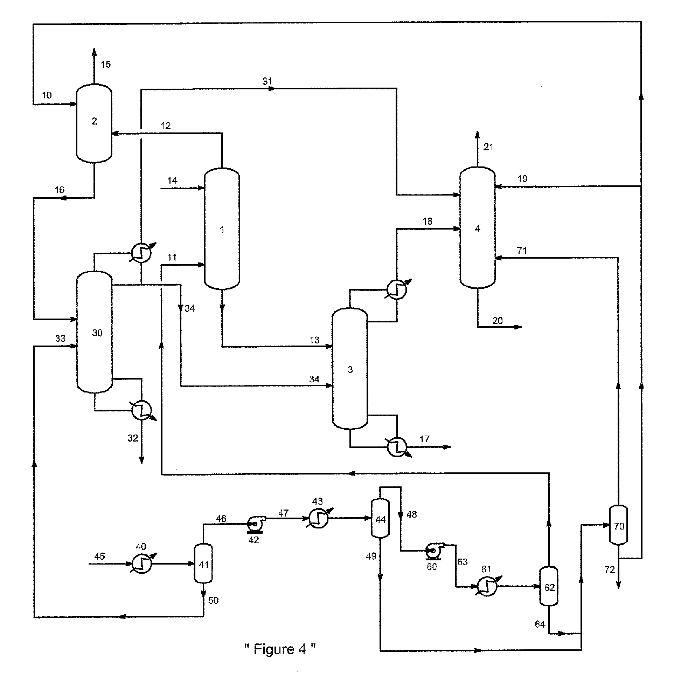

Fig 4 depicts an embodiment of the invention and derives from fig 3 by incorporation of the flash drum 70. The stream 64 and 49 are flashed to produce a gaseous phase 71 sent to the low pressure water wash column 4 and a portion of the liquid phase is used as the wash water 19 in the low pressure water wash column and as the wash water 10 in the high pressure water wash column. Liquid phase 72 containing oxygenated contaminants is optionally recycled or eliminated.

As regards alcohol dehydration, such process is described in WO-2009-098262, WO-2009-098267, WO-2009-098268 and WO-2009-098269 the content of which is incorporated in the present application. The present invention is very efficient for the purification of ethylene produced by dehydration of ethanol.

Typical weight composition of the effluent to be purified further to the ethanol dehydration are on a dry basis, the total being 100% :

| | | | |
|---|---|---|---|
| CARBON MONOXIDE | 0.01 | to | 0.1 |
| ETHANE | 0.01 | to | 0.1 |
| ETHYLENE | 95 | to | 99.75 |
| PROPYLENE | 0.0 | to | 0.01 |
| ACETALDEHYDE | 0.03 | to | 0.3 |
| ETHANOL | 0.2 | to | 2.0 |
| ISOBUTYLENE | 0.0 | to | 0.1 |
| 1-BUTENE | 0.0 | to | 0.1 |
| TRANS-2-BUTENE | 0.0 | to | 0.3 |
| CIS-2-BUTENE | 0.0 | to | 0.3 |
| 3-METHYL-1-BUTENE | 0.0 | to | 0.3 |

The proportion of water may be from 1 mole of water for one mole of ethylene to about five tons for one ton of ethylene. The above typical stream above can be purified with the process of the invention to get an ethylene stream having an acetaldehyde content of less than 5 ppm, often less than 3 ppm more often less than 2 ppm.

As regards the MTO process, such process is described in WO-2008-110526, WO-2008-110528, WO-2008-110530, WO-2009-016153, WO-2009-016154, WO-2009-016155, WO-2009-092779, WO-2009-092780, WO-2009-092781, the content of which is incorporated in the present application. The MTO process has also been described in US 2006 0235251, WO 2005 016856, US 2006 0063956, US 2006 0161035, US 6207872, US 2005 0096214, US 6953767 and US 7067095, the content of which is incorporated in the present application.

The effluent produced by a MTO process which is an hydrocarbon stream comprising oxygenated contaminants and water can be purified with the process of the invention to get an ethylene/propylene stream having an acetaldehyde content of less than 5 ppm, often less than 3 ppm more often less than 2 ppm. Said stream can be purified with the process of the invention to get an ethylene/propylene stream having a DME content of less than 5 ppm, often less than 3 ppm more often less than 2 ppm.

## Claims

1. Process for removing oxygenated contaminants and water from an hydrocarbon stream comprising :
introducing the contaminated hydrocarbon stream in a gaseous phase in an absorption zone,
contacting said hydrocarbon stream in said absorption zone at a pressure of at least 5 bars, advantageously in the range 5 to 40 bars with an alcohol capable to absorb water and oxygenated contaminants at conditions effective to produce
an overhead hydrocarbon stream having a reduced oxygenated contaminants and water content and
an absorbent bottoms stream comprising the alcohol, hydrocarbons and having an enhanced oxygenated contaminants and water content, sending the overhead of the absorption zone to a wash column (referred to as the high pressure water wash column) at a pressure of at least 5 bars, advantageously in the range 5 to 40 bars, essentially washed with water at conditions effective to produce
an overhead hydrocarbon stream having a reduced oxygenated contaminants and
an aqueous bottoms stream having an enhanced oxygenated contaminants content.

2. Process according to any one of the preceding claims wherein the alcohol is an aqueous solution comprising at least 80w% of alcohol.

3. Process according to any one of the preceding claims wherein the overhead of the high pressure water wash column is sent to a caustic wash to remove the acidic components and recovering an hydrocarbon stream essentially free of oxygenated contaminants.

4. Process according to any one of the preceding claims further comprising :
sending the bottoms stream from the absorption zone to a distillation column (referred to as the alcohol distillation column) operating at a pressure of less than 3 bars absolute and advantageously at a pressure in the range 1-3 bars absolute at conditions effective to produce
an overhead comprising essentially oxygenated contaminants and hydrocarbons, optionally treated to recover the hydrocarbons,
an alcohol bottoms stream comprising water and essentially free of hydrocarbons and oxygenated contaminants,
sending said alcohol bottoms to the process which has produced the hydrocarbon stream comprising oxygenated contaminants and water to be purified.

5. Process according to claim 4 wherein the overhead of the alcohol distillation column is sent to a wash column fed with water (referred to as the low pressure water wash column) to produce an overhead hydrocarbon stream comprising oxygenated contaminants and a bottoms aqueous stream comprising oxygenated contaminants.

6. Process according to claim 5 wherein the said overhead of the low pressure water wash column, which contains hydrocarbons, is recycled to the process which has produced the hydrocarbon stream comprising oxygenated contaminants and water to be purified.

7. Process according to any one of the preceding claims wherein the water recovered at the beginning of the course of the compression of the contaminated hydrocarbon until the entry in the absorption zone is sent to a water stripping column at low pressure equiped with a reboiler at the bottoms and a condenser-separator on top and operates at conditions effective to produce
• at the condenser separator a gaseous phase comprising oxygenated contaminants and hydrocarbons, an aqueous phase comprising alcohol, oxygenated contaminants and hydrocarbons partly sent as a reflux of said stripping column, partly sent to the alcohol distillation column, optionally mixed with the bottoms from the absorption zone,
• an essentially pure water bottoms stream.

8. Process according to claim 7 wherein the water stripping column operates at a pressure in the range 1-3 bars absolute.

9. Process according to claim 7 or 8 wherein the water recovered in the course of the compression of the contaminated hydrocarbon until the entry in the absorption zone comprises the water condensed at the beginning of said compression and the remaining water further condensed until entry in the absorption zone, the ratio of the water condensed at the beginning to the remaining water is from 40/60 to 80/20.

10. Process according to any of claims 7 to 9 wherein the gaseous phase from the condenser-separator of the water stripping column is sent to the low pressure water wash column.

11. Process according to any of claims 7 to 10 wherein the bottoms of the high pressure water wash column are sent to the water stripping column.

12. Process according to any of claims 7 to 11 wherein the remaining part of the water condensed in the course of the compression of the contaminated hydrocarbon stream to be fed in the absorption zone, after the water condensed at the beginning has been sent to the said water stripping column, is flashed in a flash drum,
• the overhead of said flash drum is sent to the low pressure water wash column,
• a part of the liquid phase is used as the wash water in the low pressure water wash column and in the high pressure water wash column,
• the remaining part of said liquid phase is purged.

13. Process according to any of claims 1 to 3 wherein the contaminated hydrocarbon stream is successively compressed and cooled in one or more steps to remove the major part of water and further fed to the absorption zone, the water recovered upon each cooling further to a compression step and upon cooling if any before the first compression step is sent to a stripping column to produce an overhead stream comprising essentially oxygenated contaminants and hydrocarbons and an essentially pure water bottoms stream.

14. Process according to any of claims 1 to 3 and claim 13 wherein the bottoms stream of the absorption zone is sent to a distillation column operating at a pressure of less than 3 bars absolute and advantageously at a pressure in the range 1-3 bars absolute to produce (i) an alcohol bottoms stream comprising water but essentially free of oxygenated contaminants recycled to the absorption zone and (ii) an overhead comprising essentially hydrocarbons and the oxygenated contaminants.

15. Process according to any one of the preceding claims wherein the hydrocarbon stream comprising oxygenated contaminants and water is the effluent produced by an alcohol dehydration to make at least an olefin.

16. Process according to claim 15 wherein ethanol is dehydrated to make ethylene.

17. Process according to anyone of claims 1 to 14 wherein the hydrocarbon stream comprising oxygenated contaminants and water is the effluent produced by a MTO process based on methanol or dimethyl ether and the alcohol of the absorption zone is methanol.
